# EUROPEAN PATENT SPECIFICATION

(11) **EP 4 398 970 B1**
(45) Date of publication and mention of the grant of the patent: **11.03.2026**
(21) Application number: 21787176.3
(22) Date of filing: 10.09.2021
(51) Int. Cl.: A61M 39/02, A61M 25/00, A61M 39/12, A61M 39/10

(54) **ANTI-INGROWTH CATHLOCK**
WACHSTUMSVERHINDERNDES CATHLOCK
CATHÉTÉRISATION ANTI-INTERPOSITION

(43) Date of publication of application: 17.07.2024
(73) Proprietor: Bard Peripheral Vascular, Inc., Franklin Lakes, NJ 07417 (US)
(72) Inventor: ANDERSEN, Christian, Kaysville, UT 84037 (US); DENSLEY, Bryon, Ray, Rochester, MN 55902 (US); FIUMEFREDDO, Diana, Salt Lake City, UT 84121 (US)
(74) Representative: Hoffmann Eitle
(86) International application number: PCT/US2021/049888
(87) International publication number: WO 2023/038632

(56) References cited:
- EP-B1- 1 986 717
- US-A- 5 129 891

## Description

### BACKGROUND

US 5, 129, 891 A discloses a connector for detachably securing an end of a tube, such as a catheter, to a fluid port of a fluid transfer assembly, such as an implantable device, the connector having a body part which defines a coupler for coupling to a coupler receiver of the transfer assembly. The connector includes a sleeve which fits within the body part and defines an aperture for compressively receiving an end of the tube which has been fitted over a fluid port of the transfer assembly. The body part is rotatable about the sleeve to facilitate coupling of the coupler and coupler receiver. The sleeve generates a compressive force to secure the tube to the fluid port. A compression insert may be inserted between the sleeve and tube to provide a higher degree of compression of the tube upon the fluid port.

EP 1986717 B1 discloses an apparatus for facilitating the replacement of an implanted catheter.

### SUMMARY

In a first aspect of the present invention, there is provided a cathlock system according to claim 1.

In a second aspect of the present invention, there is provided a system according to claim 9.

In a third aspect of the present invention, there is provided a method according to claim 12.

The dependent claims define preferred embodiments.

Briefly summarized, embodiments disclosed herein are directed to an anti-ingrowth cathlock device and associated methods thereof. The anti-ingrowth cathlock device can include a compliant, soft polymer configured to elastically deform when compressed between the resilient cathlock and one or more of the catheter, stem, port body or portions thereof. The compliant polymer portion in the deformed state expands to fill any potential gaps between the cathlock and one or more of the catheter, stem, port body or portions thereof, providing a continuous outer profile therebetween and mitigating tissue ingrowth. Advantageously, the anti-ingrowth cathlock device can facilitate explantation of the catheter and port assembly.

Disclosed herein is a cathlock system including, a port including a stem extending therefrom, a catheter defining a lumen and configured to engage the stem, a cathlock configured to encircle a portion of one or both of the stem and the catheter, and a gasket formed of a compliant material and configured to elastically transition between an undeformed state and a deformed state, the gasket in the deformed state forming a continuous outer profile between an outer surface of the cathlock and one or both of the catheter and the port.

In some embodiments, the gasket is coupled with the port body and extends annularly about the stem. In some embodiments, the gasket defines a disc shape, extending along a plane perpendicular to a longitudinal axis of the stem, an outer diameter of the gasket in the undeformed state is equal to, or less than, an outer diameter of the cathlock. In some embodiments, the outer diameter of the gasket in the deformed state is equal to or larger than the outer diameter of the cathlock. In some embodiments, the outer diameter of the gasket in the deformed state is larger than the outer diameter of the gasket in the undeformed state.

In some embodiments, the gasket is coupled to an inner surface of the cathlock and extends between a distal end and a proximal end of the cathlock. In some embodiments, the gasket further includes a flange extending radially outward from a proximal end of the gasket. In some embodiments, the flange defines a first diameter in the undeformed state, and a second diameter in the deformed state, the second diameter being larger than the first diameter. In some embodiments, the first diameter of the flange is less than an outer diameter of the cathlock, and the second diameter of the flange is equal to, or larger than the outer diameter of the cathlock.

In some embodiments, the cathlock is formed of a first material and the gasket is formed of a second material different from the first material. In some embodiments, the first material defines a first durometer and the second material defines a second durometer, the second durometer being less than the first durometer. In some embodiments, the first material is a substantially rigid material and the second material is an elastically deformable material. In some embodiments, the first material includes one of a plastic, polymer, metal, alloy, or composite, and the second material includes one of a plastic, polymer, elastomer, thermoplastic elastomer ("TPE"), rubber, or silicone rubber.

In some embodiments, the gasket is formed of a biocompatible, or inert, material and is configured to mitigate tissue ingrowth between the cathlock and one or both of the catheter and the port. In some embodiments, the cathlock system further includes a valve disposed between an outer surface of the catheter and an inner surface of the cathlock and configured to provide a fluid tight seal therebetween.

Also disclosed is a system for coupling a catheter to a subcutaneous vascular access device including, a body of the subcutaneous vascular access device having a recess disposed therein, a stem extending longitudinally from the recess, a catheter defining a lumen and configured to engage the stem, a cathlock configured to encircle a portion of one or both of the stem and the catheter, a proximal end of the cathlock configured to fit within the recess, and a gasket formed of a compliant material and configured to elastically transition between a deformed state and an undeformed state, the gasket in the deformed state forming a continuous outer profile between an outer surface of the cathlock and the catheter.

In some embodiments, the cathlock includes a flared portion disposed at a proximal end of the cathlock. In some embodiments, the flared portion co-operates with an outer surface of the body to define a continuous outer profile when the proximal end of the cathlock is disposed within the recess. In some embodiments, the gasket is coupled to an inner surface of the cathlock and extends between a distal end and the proximal end of the cathlock. In some embodiments, the gasket is formed integrally with the cathlock. In some embodiments, the gasket is formed as a separate structure and coupled with the cathlock with an interlocking fit, interference fit, press-fit, or snap fit engagement, adhesive, bonding, or welding.

In some embodiments, the cathlock is formed of a first material and the gasket is formed of a second material different from the first material. In some embodiments, the first material defines a first durometer and the second material defines a second durometer, the second durometer being less than the first durometer. In some embodiments, the cathlock system further includes a valve disposed between an outer surface of the catheter and an inner surface of the cathlock and configured to provide a fluid tight seal therebetween.

Also disclosed a method of coupling a catheter to a stem of a port including, urging a proximal portion of the catheter over the stem of the port, slidably engaging a cathlock over the proximal portion of the catheter, elastically deforming a gasket to a deformed state to provide a continuous outer profile between an outer surface of the cathlock and one or both of the catheter and the port.

In some embodiments, the gasket is coupled with the port body and defines a first outer diameter in the deformed state and a second outer diameter in an undeformed state, the first outer diameter being greater than the second outer diameter and equal to, or greater than, an outer diameter of the cathlock. In some embodiments, the gasket is coupled to an inner surface of the cathlock and extends between a distal end and a proximal end of the cathlock. In some embodiments, the method further includes compressing a flange, between the cathlock and the port, the flange extending radially outward from a proximal end of the gasket.

In some embodiments, the outer diameter of the flange in the deformed state is equal to, or larger than, the outer diameter of the cathlock. In some embodiments, the cathlock is formed of a first material and the gasket is formed of a second material different from the first material. In some embodiments, the first material defines a first durometer and the second material defines a second durometer, the second durometer being less than the first durometer. In some embodiments, the first material is a substantially rigid material and the second material is an elastically deformable material. In some embodiments, the method further includes a valve disposed between an outer surface of the catheter and an inner surface of the cathlock and configured to provide a fluid tight seal therebetween.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a plan view of a port, catheter, and cathlock assembly of a vascular access system, in accordance with embodiments disclosed herein.
FIG. 1B shows a cross-section view of a port, catheter, and cathlock assembly of a vascular access system, in accordance with embodiments disclosed herein.
FIG. 2A show a plan view of a port and catheter assembly including an anti-ingrowth cathlock, in accordance with embodiments disclosed herein.
FIG. 2B shows a cross-section view of a port and catheter assembly including an anti-ingrowth cathlock, in accordance with embodiments disclosed herein.
FIG. 2C shows an exploded perspective view of a port and catheter assembly including an anti-ingrowth cathlock, in accordance with embodiments disclosed herein.
FIGS. 2D-2F show close up detail of a gasket of an anti-ingrowth cathlock, in accordance with embodiments disclosed herein.
FIGS. 3A-3B show perspective views of an anti-ingrowth cathlock, in accordance with embodiments disclosed herein.
FIGS. 4A-4C show an exemplary method of use for an anti-ingrowth cathlock, in accordance with embodiments disclosed herein.
FIG. 5A shows a perspective view of a port, catheter and anti-ingrowth cathlock, in accordance with embodiments disclosed herein.
FIG. 5B shows a plan view of a port, catheter and anti-ingrowth cathlock, in accordance with embodiments disclosed herein.
FIG. 5C shows a perspective view of an anti-ingrowth cathlock, in accordance with embodiments disclosed herein.
FIG. 5D shows a cross-section view of a port, catheter and anti-ingrowth cathlock, in accordance with embodiments disclosed herein.
FIGS. 6A-6C show an exemplary method of use for an anti-ingrowth cathlock, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

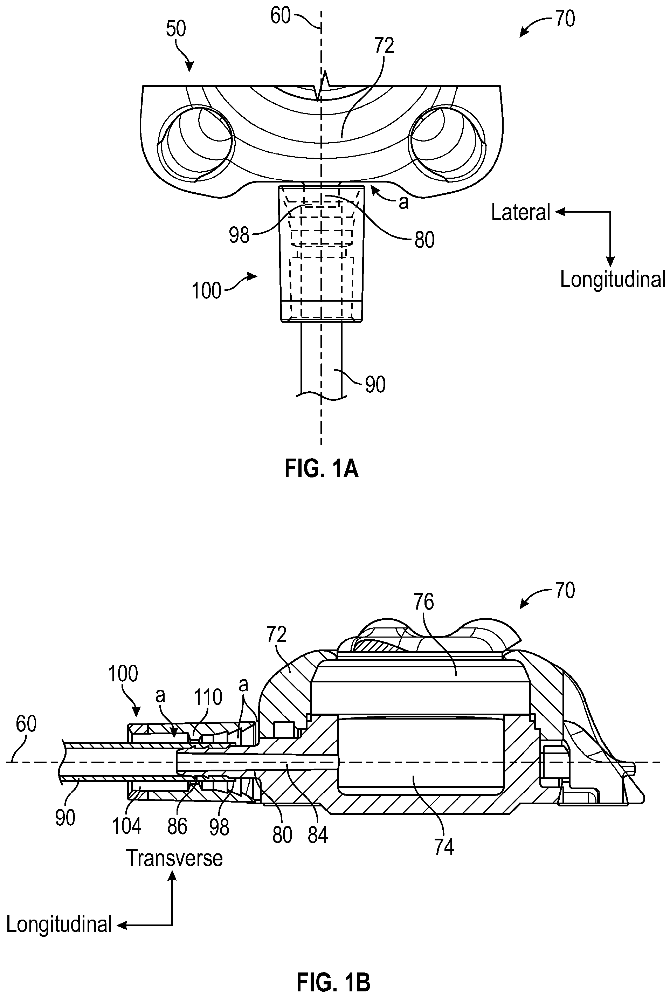

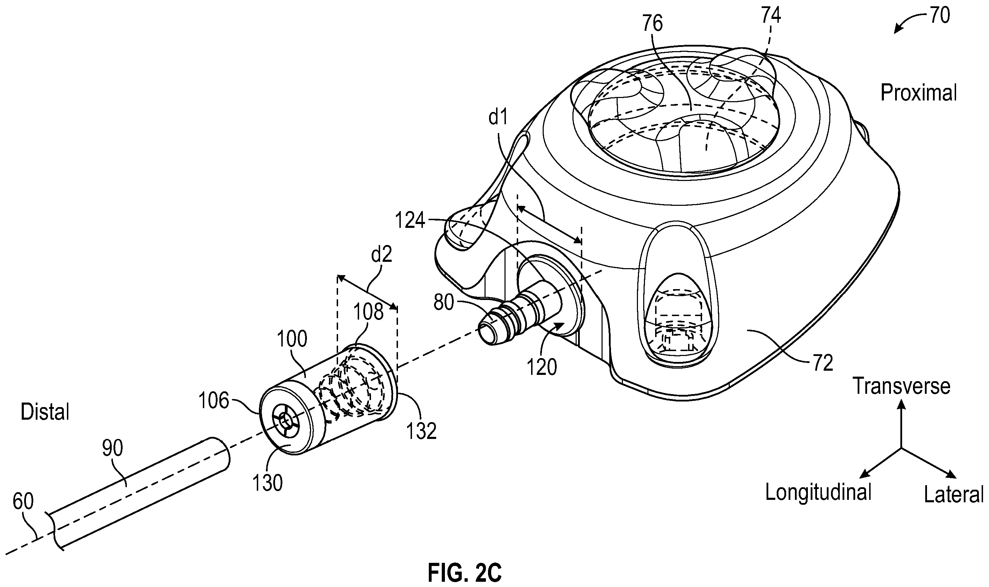

To assist in the description of embodiments described herein, as shown in FIGS. 1A-1B, 2C a longitudinal axis extends substantially parallel to an axial length of the catheter. A lateral axis extends normal to the longitudinal axis, and a transverse axis extends normal to both the longitudinal and lateral axes. As used herein, a horizontal plane extends along the lateral and longitudinal axes. A vertical plane extends normal to the horizontal plane.

Unless defined otherwise, all technical and scientific terms used herein have the same meaning as commonly understood by those of ordinary skill in the art.

FIGS. 1A-1B show an exemplary vascular access system 50 generally including a port 70, catheter 90 and a cathlock device ("cathlock") 100. The cathlock 100 can be configured to secure the catheter 90 to a stem 80 of the port 70, or similar medical device. More specifically, the cathlock 100 can slidably engage an outer surface of the catheter 90 and can compress a portion of the catheter 90 on to the stem 80 of the port 70 to provide a fluid-tight engagement therebetween.

In an embodiment, the catheter 90 can include a catheter body 92 defining a catheter lumen 94 and extending between a distal tip and a proximal end 98. In an embodiment, the proximal end 98 of the catheter 90 can be coupled to the stem 80 by urging the catheter 90 axially thereover. In an embodiment, the proximal end 98 of the catheter 90 can be trimmable prior to coupling the catheter 90 with the stem 80. In an embodiment, the catheter 90 can be formed of a flexible or compliant material configured to elastically deform radially outwards and engage the stem 80 in an interference fit. In an embodiment, the catheter 90 can be formed of a plastic, polymer, elastomer, thermoplastic elastomer ("TPE"), rubber, silicone rubber, or the like.

In an embodiment, the port 70 can generally include a body 72 defining a reservoir 74 that is in fluid communication with a lumen 84 of the stem 80. The port 70 can further include a needle penetrable septum 76 configured to provide access to the reservoir 74. In an embodiment, an access needle can extend percutaneously, through the needle-penetrable septum 76 and into the reservoir 74 to provide fluid communication therewith. As will be appreciated, the port 70 is an exemplary medical device in fluid communication with the stem 80 and embodiments described herein can be used with various medical devices that include a rigid stem 80 coupled to a compliant catheter 90.

In an embodiment, the cathlock 100 can define a cathlock lumen 104, extending longitudinally, and configured to receive one or both of a portion of the catheter 90 and a portion of the stem 80 therein. The cathlock 100 can slidably engage an outer surface of the catheter 90 and compress a portion of the catheter 90 onto the stem 80. The cathlock 100 can engage the catheter 90/stem 80 assembly in an interference fit, press-fit, or snap-fit engagement. In an embodiment, the cathlock 100 can be formed of a resilient or substantially rigid material, defining a first durometer, and can include a plastic, polymer, metal, alloy, composite, combinations thereof, or the like.

In an embodiment, the cathlock 100 can include a rim 110 or similar structure extending radially inward from an inner surface of the cathlock lumen 104. The rim 110 can be configured to engage a barb 86 or similar structure disposed on the stem 80. With the catheter 90 disposed over the barb 86 of the stem 80, the rim 110 can engage the barb 86 in an interference fit, press-fit, or snap-fit engagement, with a portion of the catheter 90 disposed therebetween.

As shown in FIGS. 1A-1B, due to the tolerances in the manufacturing of one or more of the cathlock 100, catheter 90, or port 70, gaps (*a*) can occur between one or more of the cathlock 100, catheter 90, or port 70, or combinations thereof. When placed subcutaneously, tissues can grow into these gaps (*a*) binding and encapsulating the cathlock 100, catheter 90, port 70 assembly. As such, explantation of the cathlock 100, catheter 90, port 70 assembly can be time consuming, requiring additional cutting, forming scar tissue, and increasing recovery times.

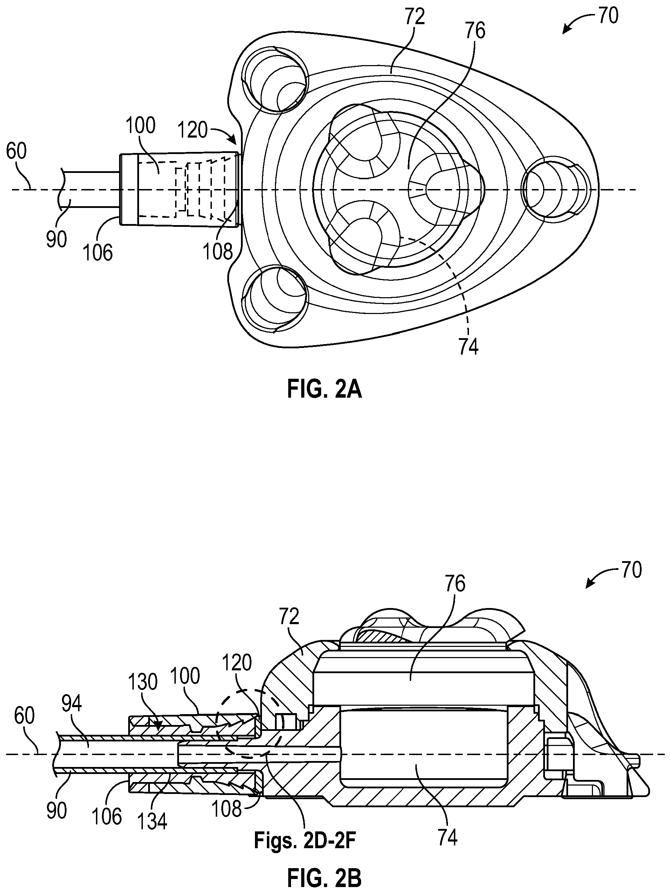

In an embodiment, as shown in FIGS. 2A-2C, the anti-ingrowth cathlock 100 can include one or more gaskets, for example a first, port gasket 120 and a second, cathlock gasket 130. The gaskets 120, 130 can be formed of a compliant material, defining a second durometer, and can include a plastic, polymer, elastomer, rubber, silicone rubber, thermoplastic elastomer ("TPE"), combinations thereof, or the like. The second durometer can be less, or softer, than the first durometer. In an embodiment, the gasket 120, 130 can be formed of a biocompatible material. In an embodiment, the gasket 120, 130 can be formed of an inert material.

In an embodiment, the gaskets 120, 130 can extend about a central longitudinal axis 60 and can be configured to provide a continuous outer surface between one or more of the catheter 90, port 70, and the cathlock 100. As used herein, a "continuous outer profile" can include a substantially even surface or convex surface extending between one or more of the catheter 90, port 70, and the cathlock 100. In an embodiment, the gaskets 120, 130 can be formed as a separate structure and coupled with one of the cathlock 100, stem 80, or port body 72 in an interference fit engagement, press-fit, or snap fit engagement, or the like. In an embodiment, one of the cathlock 100, stem 80, or port body 72 can include a recess configured to receive a portion of the gasket 120, 130 therein and retain the gasket 120, 130 in an interlocking fit, interference fit, press-fit, or snap fit engagement, or the like. In an embodiment, the gasket 120, 130 can coupled with one of the cathlock 100, stem 80, or port body 72 with adhesive, bonding, welding, or the like. In an embodiment, the gasket 120, 130 can be formed integrally with the cathlock 100, for example in a two shot injection mold process, or the like.

In an embodiment, as shown in FIG. 2C, a first, or port, gasket 120 can substantially define a disc shape extending along a plane extending perpendicular to the central longitudinal axis 60. The first gasket 120 can include a lumen 124 configured to receive a portion of the stem 80 therethrough. In an embodiment, an outer diameter (*d1*) of the first gasket 120 can be equal to or less than an outer diameter (d2) of the cathlock 100.

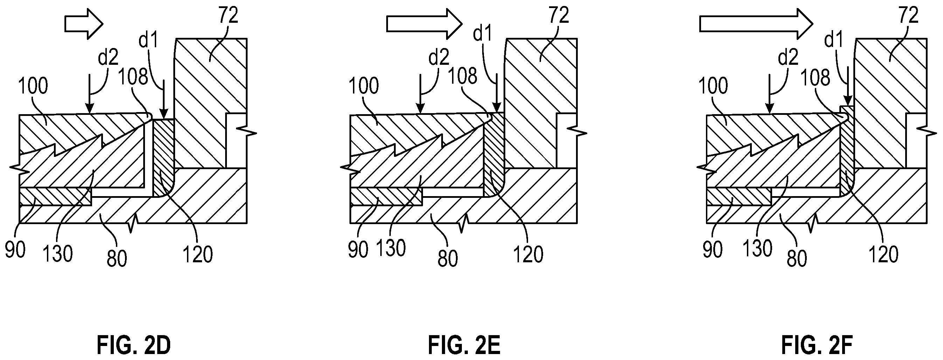

In an exemplary method of use, as shown in FIGS. 2B-2F, a first gasket 120 can be disposed over the port stem 80 (FIG. 2C). The catheter 90 can be urged axially over the stem 80 of the port 70, to engage the stem 80 in an interference fit. The cathlock 100 can be slidably engaged over the catheter 90 to compress a proximal portion of the catheter 90 on to the stem 80. The cathlock 100 can be urged axially until a proximal end 108 of the cathlock 100 compresses the first gasket 120 against the port body 72, transitioning the first gasket 120 from the undeformed state (FIG. 2D) to a deformed state (FIGS. 2E-2F). In an embodiment, the cathlock 100 can compress the first gasket 120 along a first axis (e.g. the longitudinal axis) to elastically deform the first gasket 120 along a second axis extending at an angle thereto (e.g. the lateral axis, transverse axis, or an axis extending therebetween) and transition the first gasket 120 from the undeformed state to the deformed state. In an embodiment, the cathlock 100 can elastically deform the first gasket 120 from the deformed state, until an outer diameter (*d1*) of the first gasket 120 is equal to (FIG. 2E) or larger than (FIG. 2F) an outer diameter (*d2*) of the cathlock 100 in the deformed state.

In an embodiment, the first gasket 120 in the deformed state can form a continuous outer profile between the cathlock 100 and the port body 72. For example, by compressing the first gasket 120 along a first axis, and transitioning the first gasket 120 from the undeformed state to the deformed state, an outer diameter of the first gasket 120 can expand along a second axis to fill a gap (*a*)*.* An outer surface of the gasket 120 can expand to align with an outer surface of the cathlock 100 providing a level surface therebetween (FIG. 2E). In an embodiment, the outer surface of the gasket 120 can expand to extend radially outwards of an outer surface of the cathlock 100 providing a convex surface therebetween (FIG. 2E). In an embodiment, the outer surface of the gasket 120 can expand to, or slightly less than, an outer surface of the cathlock 100. As such, although the first gasket 120 in the deformed state forms a slightly concave surface therebetween the surface is still considered substantially continuous since a depth of the gap (*a*) (i.e. perpendicular to the longitudinal axis) has been reduced to substantially less than double the width of the gap (*a*)*.*

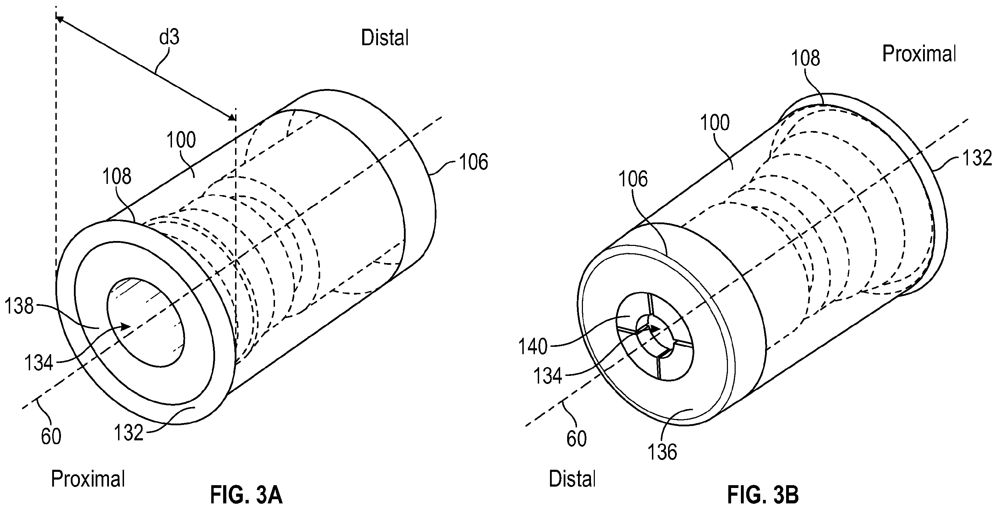

In an embodiment, as shown in FIGS. 2B, 2D-2F, and 3A-3B, a second gasket 130, or cathlock gasket 130, can be disposed on a portion of the cathlock 100, for example on an inner surface of the cathlock lumen 104. In an embodiment, the second gasket 130 can extend from a distal end 106 to a proximal end 108 of the cathlock 100. In an embodiment, the second gasket 130 can define a lumen 134 extending axially and configured to receive one or more of the catheter 90, stem 80, or portions thereof, therethrough.

In an embodiment, a diameter of the gasket lumen 134 can be equal to or less than an outer diameter of the catheter 90. In an embodiment, the lumen 134 of the second gasket 130 can elastically deform radially outward from an undeformed state to a deformed state to receive the catheter 90 therethrough and fit tightly thereon. As such, the second gasket 130 can form a continuous outer profile between the catheter 90 and the cathlock 100. For example, the second gasket 130 can form a surface extending perpendicular from the surface of the catheter 90 to a proximal end 106 of the cathlock 100, eliminating any gap (*a*) extending between the inner surface of the cathlock 100 and an outer surface of the catheter 90. In an embodiment, the second gasket 130 can include a valve 140 disposed at a distal end 136 thereof, and configured to provide a seal between the second gasket 130 and the outer surface of the catheter 90 to provide a continuous outer profile therebetween.

In an embodiment, a proximal end 138 of the second gasket 130 can include a flange 132 extending radially therefrom. In an embodiment, the flange 132 can define an outer diameter (*d3*). The outer diameter (*d3*) of the flange 132 can be less than, equal to, or larger than an outer diameter (*d2*) of the cathlock 100. In an embodiment, the flange 132 can be compressed between the cathlock 100 and one or both of the port body 72 and the first gasket 120 to transition the flange from an undeformed state to a deformed state and provide a continuous outer profile therebetween, as described herein.

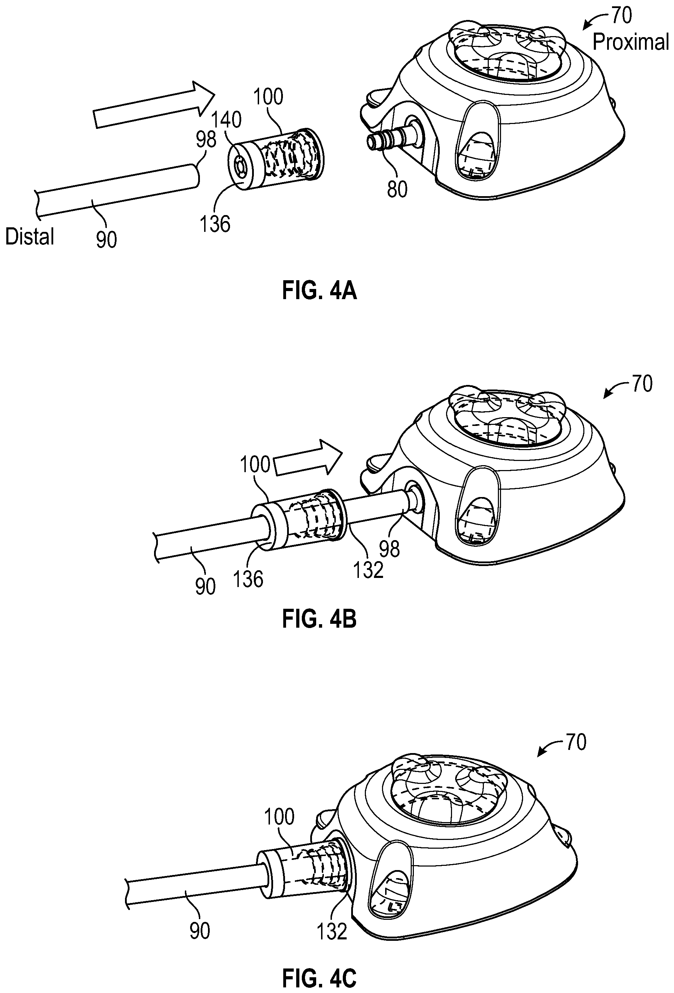

In an exemplary method of use, as shown in FIGS 4A-4C, a catheter 90 can be urged axially to engage the stem 80 in an interference fit. The cathlock 100 can then be urged axially over a proximal portion of the catheter 90, disposed over the stem 80. In an embodiment, a distal end 136 of the gasket 130 can transition to a deformed state by stretching radially outward, forming a tight seal, and providing a continuous outer profile, between the catheter 90 and the cathlock 100. In an embodiment, a valve 140 disposed at a distal end 136 of the gasket 130 can form a continuous outer profile between the catheter 90 and the cathlock 100.

Urging the cathlock 100 axially can compress one or both of a proximal end 138 of the second gasket 130 and the flange 132 against the port body 72. The cathlock 100 can elastically deform one or both of a proximal end 138 of the second gasket 130 and the flange 132 from an undeformed state to a deformed state to form a continuous outer profile between the cathlock 100 and the port body 72, as described herein. In an embodiment, the second gasket 130 can engage a first gasket 120 coupled to the port 70. The second gasket 130 and the first gasket 120 can co-operate to form a continuous outer profile between the cathlock 100 and the port body 72.

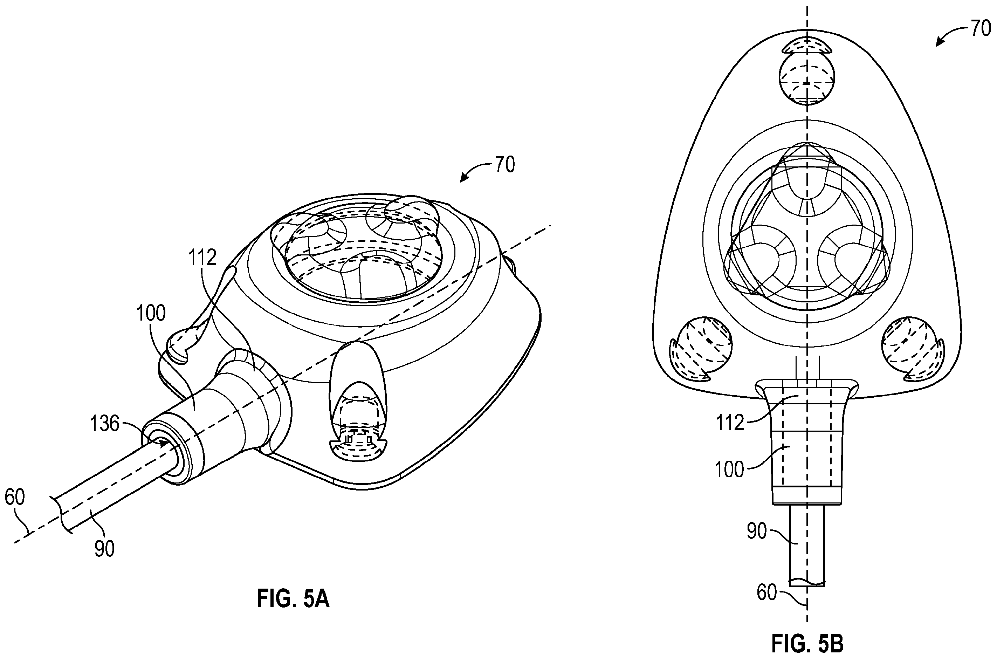

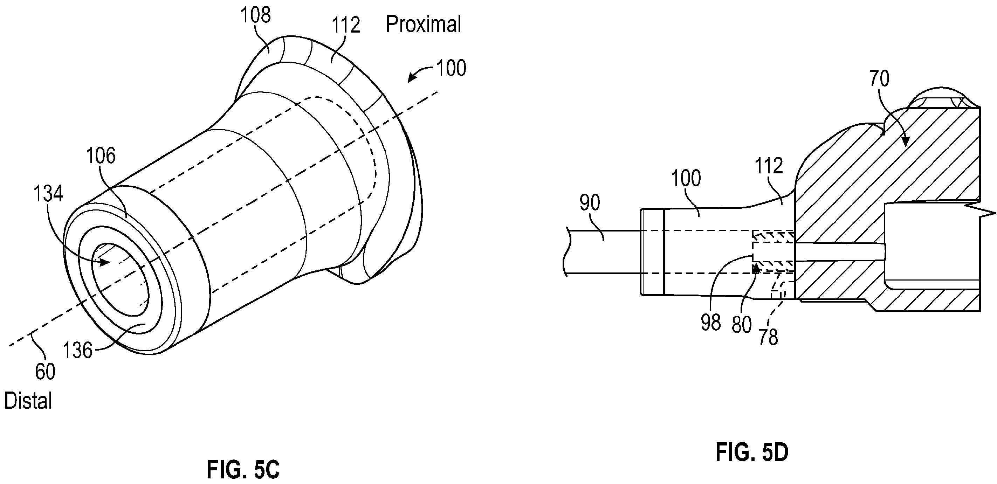

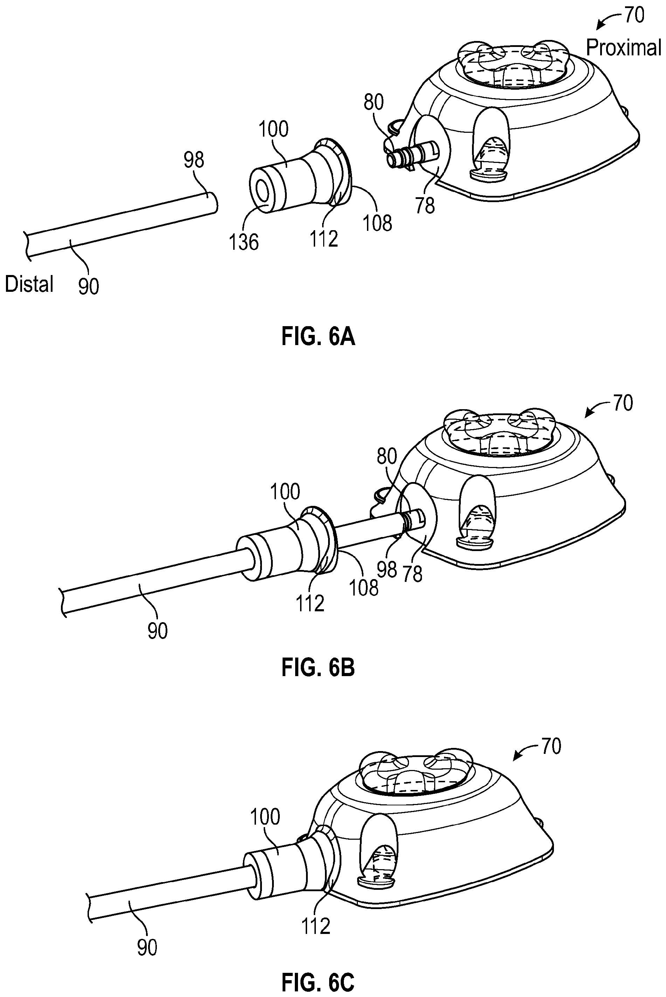

In an embodiment, as shown in FIGS. 5A-6C, a proximal end 108 of the cathlock 100 can include a flared portion 112. The flared portion 112 can be configured to engage a recess 78 disposed in the port body 72. The flared portion 112 can extend annularly about a portion of the cathlock 100, about the central longitudinal axis 60. The recess 78 can extend annularly about a portion of the stem 80, about the central longitudinal axis 60. The outer surface of the flared portion 112 can co-ordinate with an outer surface of the port body 72 to define a continuous outer profile (FIG. 5A).

In an exemplary method of use, as shown in FIGS 6A-6C, a catheter 90 can be urged axially to engage the stem 80 in an interference fit. The cathlock 100 can then be urged axially over a proximal portion of the catheter 90, disposed over the stem 80. In an embodiment, a distal end 136 of the gasket 130 can form a continuous outer profile between the catheter 90 and the cathlock 100. In an embodiment, a valve 140 disposed at a distal end 136 of the gasket 130 can form a continuous outer profile between the catheter 90 and the cathlock 100.

Urging the cathlock 100 axially can engage a proximal end 108 of the cathlock 100 within the recess 78 of the port 70. In an embodiment, the port 70 can further include a first gasket 120 disposed about the stem 80 and disposed between the cathlock 100 and the port body 72, as described herein. As shown in FIG. 6C, an outer profile of the flared portion 112 can co-ordinate with and outer profile of the port body 72 to define a continuous outer surface therebetween and can mitigate tissue ingrowth between the port body 72 and the cathlock 100.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A cathlock system for a subcutaneous vascular access device, comprising:
a port (70) including a stem (80) extending therefrom;
a catheter (90) defining a lumen (94) and configured to engage the stem;
a cathlock (100) configured to encircle a portion of one or both of the stem (80) and the catheter (90);
a cathlock gasket (130) integral with or coupled to the cathlock, the cathlock gasket extending from a distal end to a proximal end of the cathlock (100); and
a disc-shaped port gasket (120) extending along a plane perpendicular to a longitudinal axis of the stem (80),
wherein each of the port gasket (120) and the cathlock gasket (130) are formed of a compliant material and configured to elastically transition between an undeformed state and a deformed state having a continuous outer profile between an outer surface of the cathlock (100) and one or both of the catheter (90) and the port (70).

2. The cathlock system according to claim 1, wherein the port gasket (120) is coupled with the port body (72) and extends annularly about the stem (80).

3. The cathlock system according to any one of claims 1-2, wherein an outer diameter of the port gasket (120) in the undeformed state is equal to, or less than, an outer diameter of the cathlock (100).

4. The cathlock system according to claim 3, wherein the outer diameter of the port gasket (120) in the deformed state is equal to or larger than the outer diameter of the cathlock (100).

5. The cathlock system according to any one of claims 1-4, wherein the outer diameter of the port gasket (120) in the deformed state is larger than the outer diameter of the port gasket (120) in the undeformed state.

6. The cathlock system according to claim 1, wherein the cathlock gasket (130) further includes a flange (132) extending radially outward from a proximal end of the cathlock gasket (130), optionally
wherein the flange (132) defines a first diameter in the undeformed state, and a second diameter in the deformed state, the second diameter being larger than the first diameter, further optionally
wherein the first diameter of the flange (132) is less than an outer diameter of the cathlock (100), and the second diameter of the flange (132) is equal to, or larger than the outer diameter of the cathlock (100).

7. The cathlock system according to any one of claims 1-6, wherein the cathlock (100) is formed of a first material and each of the cathlock gasket (130) and the port gasket (120) is formed of the compliant material as a second material different from the first material, optionally
wherein the first material defines a first durometer and the second material defines a second durometer, the second durometer being less than the first durometer, and/or
wherein the first material is a substantially rigid material and the second material is an elastically deformable material, and/or
wherein the first material includes one of a plastic, polymer, metal, alloy, or composite, and the second material includes one of a plastic, polymer, elastomer, rubber, thermoplastic elastomer, or silicone rubber.

8. The cathlock system according to any one of claims 1-7, wherein each of the cathlock gasket (130) and the port gasket (110) is formed of a biocompatible material, and wherein the cathlock (100) is configured to mitigate tissue ingrowth between the cathlock (100) and one or both of the catheter (90) and the port (70), and/or
the port gasket (110) further including a valve disposed between an outer surface of the catheter (90) and an inner surface of the cathlock (100) configured to provide a fluid tight seal therebetween.

9. A system for coupling a catheter to a subcutaneous vascular access device, the subcutaneous vascular access device being a port, the system comprising:
the cathlock system of any of claims 1 to 8,
a body (72) of the port (70) having a recess (78) disposed therein; and
the stem (80) extending longitudinally from the recess (78).

10. The system according to claim 9, wherein the cathlock (100) includes a flared portion (112) disposed at a proximal end of the cathlock (100), optionally
wherein the flared portion (112) co-operates with an outer surface of the body to define a continuous outer profile when the proximal end of the cathlock (100) is disposed within the recess (78).

11. The system according to any one of claims 9-10, wherein the cathlock gasket (130) is formed integrally with the cathlock (100), or
wherein the cathlock gasket (130) is formed as a separate structure and coupled with the cathlock (100) with an interlocking fit, interference fit, press-fit, or snap fit engagement, adhesive, bonding, or welding.

12. A method of coupling a catheter (90) to a stem (80) of a port (70), comprising:
urging a proximal portion of the catheter (90) over the stem (80) of the port (70);
slidably engaging a cathlock (100) over the proximal portion of the catheter (90);
elastically deforming each of: i) a cathlock gasket (130) integral with the cathlock (100) or coupled to the cathlock (100), extending from a distal end to a proximal end of the cathlock (100), and ii) a disc-shaped port gasket (120) extending along a plane perpendicular to a longitudinal axis of the stem (80), to a deformed state to provide a continuous outer profile between an outer surface of the cathlock (100) and one or both of the catheter and the port (70).

13. The method according to claim 12, wherein the port gasket (120) is coupled with the port body (72) and defines a first outer diameter in the deformed state and a second outer diameter in an undeformed state, the first outer diameter being greater than the second outer diameter and equal to, or greater than, an outer diameter of the cathlock, optionally
further including compressing a flange (132), between the cathlock (100) and the port (72), the flange (132) extending radially outward from a proximal end of the cathlock gasket (130), further optionally
wherein the outer diameter of the flange (132) in the deformed state is equal to, or larger than, the outer diameter of the cathlock (100).

14. The method according to any one of claims 12-13, wherein the cathlock is formed of a first material and each of the cathlock (100) and the port gasket (120) is formed of a second material different from the first material, optionally
wherein the first material defines a first durometer and the second material defines a second durometer, the second durometer being less than the first durometer, further optionally
wherein the first material is a substantially rigid material and the second material is an elastically deformable material.

15. The method according to any one of claims 12-14, the port gasket (120) further including a valve (140) disposed between an outer surface of the catheter (90) and an inner surface of the cathlock (100) configured to provide a fluid tight seal therebetween.

## Patentansprüche

1. Cathlocksystem für eine subkutane Gefäßzugangsvorrichtung, umfassend:
einen Port (70), der einen Schaft (80) einschließt, der sich davon erstreckt;
einen Katheter (90), der ein Lumen (94) definiert und dazu konfiguriert ist, den Schaft in Eingriff zu nehmen;
einen Cathlock (100), der dazu konfiguriert ist, einen Abschnitt von einem oder beidem von dem Schaft (80) und dem Katheter (90) einzukreisen;
eine Cathlockdichtung (130), die einstückig mit dem Cathlock ist oder an diesen gekoppelt ist, wobei sich die Cathlockdichtung von einem distalen Ende zu einem proximalen Ende des Cathlocks (100) erstreckt; und
eine scheibenförmige Portdichtung (120), die sich entlang einer Ebene senkrecht zu einer Längsachse des Schafts (80) erstreckt,
wobei jede von der Portdichtung (120) und der Cathlockdichtung (130) aus einem nachgiebigen Material gebildet ist und dazu konfiguriert ist, zwischen einem unverformten Zustand und einem verformten Zustand, der ein kontinuierliches Außenprofil zwischen einer Außenfläche des Cathlocks (100) und einem oder beiden des Katheters (90) und des Ports (70) aufweist, elastisch überzugehen.

2. Cathlocksystem nach Anspruch 1, wobei die Portdichtung (120) mit dem Portkörper (72) gekoppelt ist und sich ringförmig um den Schaft (80) herum erstreckt.

3. Cathlocksystem nach einem der Ansprüche 1-2, wobei ein Außendurchmesser der Portdichtung (120) in dem unverformten Zustand gleich oder kleiner als ein Außendurchmesser des Cathlocks (100) ist.

4. Cathlocksystem nach Anspruch 3, wobei der Außendurchmesser der Portdichtung (120) in dem verformten Zustand gleich oder größer als der Außendurchmesser des Cathlocks (100) ist.

5. Cathlocksystem nach einem der Ansprüche 1-4, wobei der Außendurchmesser der Portdichtung (120) in dem verformten Zustand größer als der Außendurchmesser der Portdichtung (120) in dem unverformten Zustand ist.

6. Cathlocksystem nach Anspruch 1, wobei die Cathlockdichtung (130) weiter einen Flansch (132) einschließt, der sich radial nach außen von einem proximalen Ende der Cathlockdichtung (130) erstreckt, wobei optional
der Flansch (132) in dem unverformten Zustand einen ersten Durchmesser und in dem verformten Zustand einen zweiten Durchmesser definiert, wobei der zweite Durchmesser größer als der erste Durchmesser ist, wobei weiter optional
der erste Durchmesser des Flanschs (132) kleiner als ein Außendurchmesser des Cathlocks (100) ist und der zweite Durchmesser des Flanschs (132) gleich oder größer als der Außendurchmesser des Cathlocks (100) ist.

7. Cathlocksystem nach einem der Ansprüche 1-6, wobei der Cathlock (100) aus einem ersten Material gebildet ist und jede von der Cathlockdichtung (130) und der Portdichtung (120) aus dem nachgiebigen Material als ein zweites Material gebildet ist, das sich von dem ersten Material unterscheidet, wobei optional
das erste Material einen ersten Härtegrad definiert und das zweite Material einen zweiten Härtegrad definiert, wobei der zweite Härtegrad kleiner als der erste Härtegrad ist, und/oder
wobei das erste Material ein im Wesentlichen starres Material ist und das zweite Material ein elastisch verformbares Material ist und/oder
wobei das erste Material eines von einem Kunststoff, Polymer, Metall, einer Legierung oder einem Verbundwerkstoff einschließt und das zweite Material eines von einem Kunststoff, Polymer, Elastomer, Gummi, thermoplastischen Elastomer oder Silikongummi einschließt.

8. Cathlocksystem nach einem der Ansprüche 1-7, wobei jede von der Cathlockdichtung (130) und der Portdichtung (110) aus einem biokompatiblen Material gebildet ist und wobei der Cathlock (100) dazu konfiguriert ist, ein Einwachsen von Gewebe zwischen dem Cathlock (100) und einem oder beiden des Katheters (90) und des Ports (70) zu mindern, und/oder
die Portdichtung (110) weiter ein Ventil einschließt, das zwischen einer Außenfläche des Katheters (90) und einer Innenfläche des Cathlocks (100) angeordnet ist und dazu konfiguriert ist, eine fluiddichte Abdichtung zwischen diesen bereitzustellen.

9. System zum Koppeln eines Katheters an eine subkutane Gefäßzugangsvorrichtung, wobei die subkutane Gefäßzugangsvorrichtung ein Port ist, das System umfassend:
das Cathlocksystem nach einem der Ansprüche 1 bis 8,
einen Körper (72) des Ports (70), der eine darin angeordnete Aussparung (78) aufweist; und
wobei sich der Schaft (80) längs von der Aussparung (78) erstreckt.

10. System nach Anspruch 9, wobei der Cathlock (100) einen aufgeweiteten Abschnitt (112) einschließt, der an einem proximalen Ende des Cathlocks (100) angeordnet ist, wobei optional der aufgeweitete Abschnitt (112) mit einer Außenfläche des Körpers zusammenwirkt, um ein kontinuierliches Außenprofil zu definieren, wenn das proximale Ende des Cathlocks (100) innerhalb der Aussparung (78) angeordnet ist.

11. System nach einem der Ansprüche 9-10, wobei die Cathlockdichtung (130) einstückig mit dem Cathlock (100) gebildet ist oder
wobei die Cathlockdichtung (130) als eine separate Struktur ausgebildet ist und mit dem Cathlock (100) durch eine formschlüssige Verbindung, eine Interferenzpassung, einen Presssitz oder eine Schnappverbindung, durch Kleben, Bonding oder Schweißen gekoppelt ist.

12. Verfahren zum Koppeln eines Katheters (90) an einen Schaft (80) eines Ports (70), umfassend:
Zwängen eines proximalen Abschnitts des Katheters (90) über den Schaft (80) des Ports (70); gleitendes Ineingriffbringen eines Cathlocks (100) über den proximalen Abschnitt des Katheters (90);
elastisches Verformen von jeder von: i) einer Cathlockdichtung (130), die einstückig mit dem Cathlock (100) gebildet oder an den Cathlock (100) gekoppelt ist, die sich von einem distalen Ende zu einem proximalen Ende des Cathlocks (100) erstreckt, und ii) einer scheibenförmige Portdichtung (120), die sich entlang einer Ebene senkrecht zu einer Längsachse des Schafts (80) erstreckt, in einen verformten Zustand, um ein kontinuierliches Außenprofil zwischen einer Außenfläche des Cathlocks (100) und einem oder beiden von dem Katheter und dem Port (70) bereitzustellen.

13. Verfahren nach Anspruch 12, wobei die Portdichtung (120) mit dem Portkörper (72) gekoppelt ist und in dem verformten Zustand einen ersten Außendurchmesser und in einem unverformten Zustand einen zweiten Außendurchmesser definiert, wobei der erste Außendurchmesser größer als der zweite Außendurchmesser und gleich oder größer als ein Außendurchmesser des Cathlocks ist, optional
weiter einschließend Komprimieren eines Flanschs (132) zwischen dem Cathlock (100) und dem Port (72), wobei sich der Flansch (132) radial nach außen von einem proximalen Ende der Cathlockdichtung (130) erstreckt, wobei weiter optional
der Außendurchmesser des Flanschs (132) in dem verformten Zustand gleich oder größer als der Außendurchmesser des Cathlocks (100) ist.

14. Verfahren nach einem der Ansprüche 12-13, wobei der Cathlock aus einem ersten Material gebildet ist und jedes von dem Cathlock (100) und der Portdichtung (120) aus einem zweiten Material gebildet ist, das sich von dem ersten Material unterscheidet, wobei optional
das erste Material einen ersten Härtegrad definiert und das zweite Material einen zweiten Härtegrad definiert, wobei der zweite Härtegrad kleiner als der erste Härtegrad ist, wobei weiter optional
das erste Material ein im Wesentlichen starres Material ist und das zweite Material ein elastisch verformbares Material ist.

15. Verfahren nach einem der Ansprüche 12-14, wobei die Portdichtung (120) weiter ein Ventil (140) einschließt, das zwischen einer Außenfläche des Katheters (90) und einer Innenfläche des Cathlocks (100) angeordnet ist, das dazu konfiguriert ist, eine fluiddichte Abdichtung zwischen diesen bereitzustellen.

## Revendications

1. Système de verrou de cathéter pour un dispositif d'accès vasculaire sous-cutané, comprenant :
une chambre (70) incluant un embout (80) s'étendant depuis celui-ci ;
un cathéter (90) délimitant une lumière (94) et configuré pour venir en prise avec l'embout ;
un verrou de cathéter (100) configuré pour encercler une partie de l'un ou les deux parmi l'embout (80) et le cathéter (90) ;
un joint de verrou de cathéter (130) solidaire du verrou de cathéter ou accouplé à celui-ci, le joint de verrou de cathéter s'étendant depuis une extrémité distale vers une extrémité proximale du verrou de cathéter (100) ; et
un joint de chambre en forme de disque (120) s'étendant le long d'un plan perpendiculaire vers un axe longitudinal de l'embout (80),
dans lequel chacun parmi le joint de chambre (120) et le joint de verrou de cathéter (130) sont formés d'un matériau souple et configurés pour passer élastiquement d'un état non déformé à un état déformé présentant un profil externe continu entre une surface externe du verrou de cathéter (100) et l'un ou les deux parmi le cathéter (90) et la chambre (70).

2. Système de verrou de cathéter selon la revendication 1, dans lequel le joint de chambre (120) est accouplé au corps de chambre (72) et s'étend de manière annulaire autour de l'embout (80).

3. Système de verrou de cathéter selon l'une quelconque des revendications 1 à 2, dans lequel un diamètre externe du joint de chambre (120) dans l'état non déformé est égal ou inférieur à un diamètre externe du verrou de cathéter (100).

4. Système de verrou de cathéter selon la revendication 3, dans lequel le diamètre externe du joint de chambre (120) dans l'état déformé est supérieur ou égal au diamètre externe du verrou de cathéter (100).

5. Système de verrou de cathéter selon l'une quelconque des revendications 1 à 4, dans lequel le diamètre externe du joint de chambre (120) dans l'état déformé est supérieur au diamètre externe du joint de chambre (120) dans l'état non déformé.

6. Système de verrou de cathéter selon la revendication 1, dans lequel le joint de verrou de cathéter (130) inclut en outre un rebord (132) s'étendant radialement vers l'extérieur depuis une extrémité proximale du joint de verrou de cathéter (130), facultativement
dans lequel le rebord (132) délimite un premier diamètre dans l'état non déformé, et un second diamètre dans l'état déformé, le second diamètre étant supérieur au premier diamètre, en outre facultativement
dans lequel le premier diamètre du rebord (132) est inférieur à un diamètre externe du verrou de cathéter (100), et le second diamètre du rebord (132) est égal ou supérieur au diamètre externe du verrou de cathéter (100).

7. Système de verrou de cathéter selon l'une quelconque des revendications 1 à 6, dans lequel le verrou de cathéter (100) est formé d'un premier matériau et chacun parmi le joint de verrou de cathéter (130) et le joint de chambre (120) est formé du matériau souple en tant que second matériau différent du premier matériau, facultativement
dans lequel le premier matériau délimite un premier duromètre et le second matériau délimite un second duromètre, le second duromètre étant inférieur au premier duromètre, et/ou
dans lequel le premier matériau est un matériau sensiblement rigide et le second matériau est un matériau élastiquement déformable, et/ou
dans lequel le premier matériau inclut l'un parmi un plastique, un polymère, un métal, un alliage ou un composite, et le second matériau inclut l'un parmi un plastique, un polymère, un élastomère, un caoutchouc, un élastomère thermoplastique ou un caoutchouc de silicone.

8. Système de verrou de cathéter selon l'une quelconque des revendications 1 à 7, dans lequel chacun parmi le joint de verrou de cathéter (130) et le joint de chambre (110) est formé d'un matériau biocompatible, et dans lequel le verrou de cathéter (100) est configuré pour atténuer une croissance tissulaire entre le verrou de cathéter (100) et l'un ou les deux parmi le cathéter (90) et la chambre (70), et/ou
le joint de chambre (110) incluant en outre une valve disposée entre une surface externe du cathéter (90) et une surface interne du verrou de cathéter (100) configurée pour fournir une étanchéité aux fluides entre celles-ci.

9. Système pour accoupler un cathéter à un dispositif d'accès vasculaire sous-cutané, le dispositif d'accès vasculaire sous-cutané étant une chambre, le système comprenant :
le système de verrou de cathéter selon l'une quelconque des revendications 1 à 8,
un corps (72) de la chambre (70) présentant un évidement (78) disposé à l'intérieur de celui-ci ; et
l'embout (80) s'étendant longitudinalement depuis l'évidement (78).

10. Système selon la revendication 9, dans lequel le verrou de cathéter (100) inclut une partie évasée (112) disposée au niveau d'une extrémité proximale du verrou de cathéter (100), facultativement
dans lequel la partie évasée (112) coopère avec une surface externe du corps pour délimiter un profil externe continu lorsque l'extrémité proximale du verrou de cathéter (100) est disposée à l'intérieur de l'évidement (78).

11. Système selon l'une quelconque des revendications 9 à 10, dans lequel le joint de verrou de cathéter (130) est formé solidairement avec le verrou de cathéter (100), ou
dans lequel le joint de verrou de cathéter (130) est formé en tant que structure séparée et accouplé au verrou de cathéter (100) avec une mise en prise par emboîtement, par ajustement serré, par ajustement par pression ou par encliquetage, un adhésif, une liaison ou un soudage.

12. Procédé d'accouplement d'un cathéter (90) à un embout (80) d'une chambre (70), comprenant :
le fait de pousser une partie proximale du cathéter (90) sur l'embout (80) de la chambre (70) ; la mise en prise coulissante d'un verrou de cathéter (100) sur la partie proximale du cathéter (90) ;
la déformation élastique de chacun parmi :
i) un joint de verrou de cathéter (130) solidaire du verrou de cathéter (100) ou accouplé au verrou de cathéter (100), s'étendant depuis une extrémité distale vers une extrémité proximale du verrou de cathéter (100), et
ii) un joint de chambre en forme de disque (120) s'étendant le long d'un plan perpendiculaire à un axe longitudinal de l'embout (80), vers un état déformé pour fournir un profil externe continu entre une surface externe du verrou de cathéter (100) et l'un ou les deux parmi le cathéter et la chambre (70).

13. Procédé selon la revendication 12, dans lequel le joint de chambre (120) est accouplé au corps de chambre (72) et délimite un premier diamètre externe dans l'état déformé et un second diamètre externe dans un état non déformé, le premier diamètre externe étant supérieur au second diamètre externe et égal ou supérieur à un diamètre externe du verrou de cathéter, facultativement
incluant en outre la compression d'un rebord (132), entre le verrou de cathéter (100) et la chambre (72), le rebord (132) s'étendant radialement vers l'extérieur depuis une extrémité proximale du joint de verrou de cathéter (130), en outre facultativement
dans lequel le diamètre externe du rebord (132) dans l'état déformé est égal ou supérieur au diamètre externe du verrou de cathéter (100).

14. Procédé selon l'une quelconque des revendications 12 à 13, dans lequel le verrou de cathéter est formé d'un premier matériau et chacun parmi le verrou de cathéter (100) et le joint de chambre (120) est formé d'un second matériau différent du premier matériau, facultativement
dans lequel le premier matériau délimite un premier duromètre et le second matériau délimite un second duromètre, le second duromètre étant inférieur au premier duromètre, en outre facultativement
dans lequel le premier matériau est un matériau sensiblement rigide et le second matériau est un matériau élastiquement déformable.

15. Procédé selon l'une quelconque des revendications 12 à 14, le joint de chambre (120) incluant en outre une valve (140) disposée entre une surface externe du cathéter (90) et une surface interne du verrou de cathéter (100) configurée pour fournir une étanchéité aux fluides entre celles-ci.
